# EUROPEAN PATENT APPLICATION

(11) **EP 3 889 249 A1**
(43) Date of publication of application: **06.10.2021**
(21) Application number: 19887598.1
(22) Date of filing: 22.11.2019
(51) Int. Cl.: C12N 1/14, C12N 1/20, A23L 33/10, C12Q 1/02, A61K 8/60, A61K 8/73, A61K 31/7008, A61K 35/74, G01N 33/50, A61P 1/00

(54) **EXOSOME AND VARIOUS USES THEREOF**

(30) Priority: 22.11.2018 KR 20180145374
(71) Applicant: Prostemics Co., Ltd., Seoul 06029 (KR)
(72) Inventor: CHOI, Eun Wook, Seoul 08098 (KR); PARK, Byung Soon, Seoul 06001 (KR)
(74) Representative: IPrime Rentsch Kaelin AG
(86) International application number: PCT/KR2019/016141
(87) International publication number: WO 2020/106099

(57) **Abstract**

The present invention relates to a novel exosome comprising glucosamine, glucosamine derivatives, or salts thereof. The exosome provided by the present invention has an excellent anti-inflammatory effect, and thus can effectively prevent, ameliorate, or treat various inflammatory diseases. The exosome also has an excellent effect of treating intestinal diseases, improving the skin, treating wounds, or treating hair loss.

## Description

### [Technical Field]

The present invention relates to a novel exosome and various uses thereof.

### [Background Art]

Cells release various membrane-type vesicles into the extracellular environment, and such releasing vesicles are commonly referred to as "extracellular vesicles (EVs)." Extracellular vesicles are also referred to as cell membrane-derived vesicles, ectosomes, shedding vesicles, microparticles, exosomes and the like.

The exosome is an endoplasmic reticulum consisting of several tens to hundreds of nanometer-sized vesicles formed of a double phospholipid membrane having the same structure as a cell membrane, and contains protein, mRNA, miRNA, and the like, which are called exosome cargo. Exosomal cargoes include a wide range of signaling factors, which are known to be cell type-specific and differently regulated according to the secreted cell environments. Exosomes are intercellular signaling mediators secreted by cells, and it is known that various cellular signals transmitted through the exosome regulate cell behavior including activation, growth, migration, differentiation, dedifferentiation, apoptosis, and necrosis of target cells.

Inflammatory bowel disease (IBD) is a condition in which abnormal chronic inflammation in the intestine repeats improvement and recurrence, and ulcerative colitis and Crohn's disease are typical IBDs. Although a clear pathogenesis of inflammatory bowel disease is not disclosed, it is known that genetic and immunological abnormalities and environmental factors such as stress and drugs are related thereto.

Ulcerative colitis is characterized by inflammation of the colonic mucosa from the rectum to the proximal part of the large intestine, and involves mucus-stained bloody stools, diarrhea in several to dozens of times, and fever in severe cases.

Crohn's disease may occur in the entire gastrointestinal tract from the oral cavity to the anus, and usually cause abdominal pain, diarrhea, general weakness, weight loss or anal pain, and in severe cases, intestinal stenosis, perforation, abscess, fistula, etc., thus deteriorating the quality of life. In some cases, repeated surgery is required.

Enteritis is inflammation of the mesentery, which is classified into viral, bacterial, fungal, parasitic, addictive and historical records depending on the causes. Further, based on pathological findings, this disease may also be classified into categorical, hemorrhagic, purulent, necrotic, and diphtheria type enteritis.

In addition, the symptoms of inflammatory bowel disease are not limited to the gastrointestinal tract, and may be a systemic disease. Due to damage to the intestinal tissue, it may cause severe food allergies and irritability, as well as immune diseases. Further, patients with the inflammatory bowel disease may also include extra-intestinal manifestations affecting the organs such as joints, kidneys, mouth, eyes, as well as mouth ulcers and uveitis.

Currently, therapeutic agents used to treat inflammatory bowel disease are related to alleviation of symptoms rather than direct treatment. In fact, in order to maintain such alleviated conditions as long as possible, immunosuppressive agents, aminosalicylic acid preparations, adrenal cortical steroid drugs, etc. are generally used, however, various side effects such as nausea, heartburn, headache, dizziness, anemia and skin rash in some of patients have been reported.

Accordingly, in order to solve the above problems, research on new therapeutic agents for inflammatory bowel disease has been actively conducted (Korean Patent Registration No. 10-1145751), but still insufficient.

### [Summary of Invention]

### [Problems to be Solved by Invention]

Accordingly, one object of the present invention is to provide a novel exosome having anti-inflammatory effects.

Another object of the present invention is to provide a composition for various uses using the exosome described above.

However, the technical problems to be solved by the present invention are not limited to the above-mentioned problems, and other problems not mentioned herein will be clearly understood by those skilled in the art from the following description.

### [Means for Solving Problems]

According to one embodiment of the present invention, there is provided an exosome containing glucosamine, a glucosamine derivative or a salt thereof.

The term "exosome" as used herein refers to a small vesicle in a membrane structure secreted from various cells. The exosome has a particle diameter of about 30 to 100 nm, and refers to vesicles that are released into the extracellular environment by fusion of polycysts with plasma membranes.

In the present invention, the exosome may include glucosamine, glucosamine derivatives, or salts thereof.

In the present invention, the glucosamine (C6H13ON5) is a kind of hexosamine, and is also called chitosamine as a representative natural amino sugar.

The derivative of glucosamine used herein is included within the scope of the present invention. The derivative of the glucosamine is one in which hydrogen of one or more hydroxy groups in the glucosamine is substituted with an acyl or alkyl group, for example, an acyl having 2 to 18 carbon atoms or a straight or side chain alkyl having 1 to 5 carbon atoms, in particular, may be substituted with an acyl group such as acetyl, propionyl, butyryl, pentanoyl, hexanoyl, heptanoyl, octanoyl, nonanoyl, decanoyl, undecanoyl, lauryl, tridecanoyl, myristyl, pentadecanoyl, palmitoyl, margaryl or stearyl, or an alkyl group such as methyl, ethyl, propyl, butyl, pentyl, isopropyl, isobutyl or secbutyl.

Further, salt forms of the glucosamine or glucosamine derivatives used herein are also included within the scope of the present invention. The salts thereof may include, for example: organic or inorganic acid salts such as hydrochloride, bromate, sulfate, phosphate, acetate, citrate, fumarate, lactate, maleate, succinate, tartrate, etc.; alkali-metal salts such as sodium salt, potassium salt, etc.; organic basic salts such as ammonium salt, trimethylamine, triethylamine, pyridine, picoline, 2,6-lutidine, ethanolamine, diethanolamine, triethanolamine, cyclohexylamine, dicyclohexylamine, etc., but it is not limited thereto.

In the present invention, the exosome may include glucosamine, a glucosamine derivative or a salt thereof, in a form of glycoprotein, glycolipid or polysaccharide containing the same.

In the present invention, the glucosamine, glucosamine derivative or salt thereof may be included in the exosome in an amount of more than 0 and 20% by weight ("wt.%") or less, more than 0 and 19 wt.% or less, more than 0 and 18 wt.% or less, more than 0 and 17 wt.% or less, more than 0 and 16 wt.% or less, more than 0 and 15 wt.% or less, more than 0 and 14 wt.% or less, more than 0 and 13 wt.% or less, more than 0 and 12 wt.% or less, more than 0 and 11 wt.% or less, more than 0 and 10 wt.% or less, more than 0 and 9 wt.% or less, more than 0 and 8 wt.% or less, more than 0 and 7 wt.% or less, more than 0 and 6 wt.% or less, more than 0 and 5 wt.% or less, 0.1 to 10 wt.%, 0.1 to 9 wt.%, 0.1 to 8 wt.%, 0.1 to 7 wt.%, 0.1 to 6 wt.%, or 0.1 to 5 wt.%, but it is not limited thereto. In the present invention, the exosome is derived from cells, wherein the cells may be prokaryotic cells or eukaryotic cells. In the present invention, the prokaryotic cells may be bacterial cells, wherein the bacteria may be gram negative or gram positive bacteria. Further, in the present invention, the eukaryotic cells may be plant cells, animal cells or fungal cells.

According to one embodiment of the present invention, the exosome may be derived from one or more microorganisms selected from the group consisting of Lactobacillus genus, Leuconostoc genus, Pediococcus genus, Lactococcus genus, Streptococcus genus, Aerococcus genus, Carnobacterium genus, Enterococcus genus, Oenococcus genus, Bifidobacterium genus, Sporolactobacillus genus, Tetragenococcus genus, Vagococcus genus, Weisella, Propionibacterium genus, Pediococcus genus, Staphylococcus genus, Peptostrepococcus genus, Bacillus genus, Micrococcus genus, Listeria genus, Escherichia genus, Debaromyces genus, Candida genus, Saccharomyces genus, Pichia genus, Torulopsis genus, Aspergillus genus, Rhizopus genus, Mucor genus, Penicillium genus, Bacteroides genus, Clostridium genus, Fusobacterium genus and Melissococcus genus.

According to one embodiment of the present invention, the exosome may be derived from one or more microorganisms selected from the group consisting of Bacillus cereus, Bacillus licheniformis, Bacillus subtilis, Bifidobacterium bifidum, Bifidobacterium infantis, Bifidobacterium longum, Bifidobacterium lactis, Bifidobacterium animalis, Enterococcus Faecium, Enterococcus Faecalis, Lactobacillus acidopilus, Lactobacillus kefirgranum, Lactobacillus kefiranofaciens, Lactobacillus kefiri, Lactobacillus alimentarius, Lactobacillus bulgaricus, Lactobacillus casei, Lactobacillus curvatus, Lactobacillus delbrukii, Lactobacillus johnsonii, Lactobacillus farciminus, Lactobacillus gasseri, Lactobacillus helveticus, Lactobacillus rhamnosus, Lactobacillus reuteri, Lactobacillus sakei, Lactococcus lactis, Lactobacillus brevis, Lactobacillus plantarum, Lactobacillus pentosus, Lactobacillus kimchi, Lactobacillus confusus, Lactobacillus curvatus, Streptococcus faecium, Streptococcus faecalis, Streptococcus agalactiae, Streptococcus mutans, Streptococcus thermophilus, Streptococcus lactis, Weissella confusa, Weissella koreensis, Weissella kimchii , Weissella cibaria, Weissella viridescens, Pediococcus acidilactici, Pediococcus pentosaceus, Pediococcus acidilactici, Pediococcus halophilus, Staphylococcus carnosus, Staphylococcus xylosus, Staphylococcus aureus, Escherichia coli, Tetragenococcus halophilus, Saccharomyces cerevisiae, Saccharomyces servazzii, Saccharomyces boulardii and Listeria monocytogens, but it is not limited thereto.

According to another embodiment of the present invention, there is provided a cell culture or cell culture medium including the exosome of the present invention.

The term "culture" as used herein means a product obtained by culturing the cell in a known liquid medium or solid medium, and is a concept in which cells are included. The term "culture medium" as used herein means a product obtained by culturing the cells in a known liquid medium or solid medium, and is a concept in which the cells are not included. That is, in the present invention, the culture medium may be a liquid product obtained by culturing predetermined cells in a medium, and then removing the cultured cells through filtration or centrifugation.

According to another embodiment of the present invention, there is provided a composition for promoting proliferation of stem cells containing the exosome provided by the present invention.

Since the exosome containing glucosamine of the present invention has a very excellent stem cell proliferation promoting ability, it is possible to promote the proliferation of stem cells in a serum-free medium instead of animal serum.

As used herein, the term "stem cell" refers to a cell having an ability to differentiate into two or more different types of cells while having a self-replicating ability as undifferentiated cells.

The stem cells of the present invention may be autologous or allogeneic stem cells, and may be derived from any type of animal including human and non-human mammals. The stem cells may be derived from adults or embryos without limitation thereof.

The stem cells of the present invention may include embryonic stem cells or adult stem cells, and are preferably adult stem cells. The adult stem cells may be mesenchymal stem cells, human tissue-derived mesenchymal stromal cells, human tissue-derived mesenchymal stem cells, multipotent stem cells or amniotic epithelial cells, and preferably mesenchymal stem cells, but it is not limited thereto. The mesenchymal stem cells may be mesenchymal stem cells derived from umbilical cord, umbilical cord blood, bone marrow, fat, muscle, nerve, skin, amniotic membrane and placenta, but it is not limited thereto.

In the present invention, the adult stem cell refers to undifferentiated cells having multipotency derived from a mammal, including a human, preferably a human adult cell, for example, may be derived from various adult cells such as bone marrow, blood, brain, skin, fat (i.e., adipose tissues or adipocytes), umbilical cord blood, umbilical cord Wharton's jelly. In the present disclosure, the "adult stem cell" may include mesenchymal stem cells derived from adult cells.

As the adult stem cells, adipose-derived stem cells which can be obtained using adipose tissues discarded in a commonly and often performed liposuction process, thereby not requiring any invasive procedure, are preferably used. The adipose-derived stem cells may be obtained from a mammal, and preferably human adipose tissue or adipose cells by any known method (for example, International Patent Publication No. WO2000/53795 and WO2006/042730), in particular, through different processes, for example, liposuction and sedimentation, enzyme treatment using collagenase, or the like, removal of suspended cells such as red blood cells by centrifugation. The adipose tissue may include brown or white tissues derived from subcutaneous, retinal, visceral, mammary gonads or other adipose tissue sites, and may be readily obtained from conventional liposuction.

According to another embodiment of the present invention, there is provided a culture medium composition for culturing stem cells containing the exosome provided by the present invention

In the present invention, the culture medium may be used without limitation as long as it is a medium well known to those skilled in the art. The medium may be artificially synthesized, and may be any commercially available medium. Examples of commercially produced media may include Dulbecco's Modified Eagle's Medium (DMEM), Minimal Essential Medium (MEM), Basal Medium Eagle (BME), RPMI 1640, F-10, F-12, α-MEM (α-Minimal Essential Medium), Glasgow's Minimal Essential Medium (G-MEM), Isocove's Modified Dulbecco's Medium (IMDM), and MEF, but it is not limited thereto.

According to another embodiment of the present invention, there is provided an anti-inflammatory composition including the exosome provided by the present invention as an active ingredient.

As used herein, the term "inflammation" refers to a phenomenon that appears as a series of defense purposes to minimize the reaction and to restore a damaged site into its original state, when cells or tissues are damaged due to any cause. Specifically, reactions in nerve and blood vessels or lymphatic vessels, fluid response and/or cellular reactions may occur, causing pain, swelling, redness, fever, etc., and eventually leading to dysfunction. Causes of inflammation may include physical factors such as trauma, frostbite, burns, radioactivity, chemical factors due to chemical materials such as acids, immunological factors caused by antibody reactions and the like. Further, inflammation may also be caused by blood vessel or hormone imbalance. Cells damaged by external stimulation secrete various biological mediators such as pro-inflammatory cytokines and chemokine, interleukine, and interferon, hence causing vasodilation and increased permeability. As a result, antibodies, complements, plasma and phagocytic cells may flock to a site of inflammation, and this phenomenon may cause erythema.

As used herein, the term "anti-inflammatory composition (anti-inflammatory agent)" refers to a drug that acts to remove an inflammation source and to reduce biological reactions and symptoms, thereby eliminating inflammation.

The exosome provided by the present invention inhibits expression or activity of the inflammatory cytokine thus having excellent anti-inflammatory effects, while being superior in safety to the human body.

In the present invention, the anti-inflammatory composition may be used to prevent, improve or treat inflammatory diseases.

It is generally known that inflammatory diseases are caused by various inflammatory cytokines. That is, inflammatory cytokines are closely associated with inflammatory diseases as follows: IL-2 (Interleukin-2), IL-1β (Interleukin-lbeta), IL-6, and TNF-α ( (Tumor necrosis factor-alpha) are related to pelvic inflammatory diseases [Clin Chem Lab Med. 2008; 46 (11): 1609-1616, Significant elevation of a Th2 cytokine, interleukin-10, in pelvic inflammatory disease; Clin Chem Lab Med. 2008; 46 (7): 997-1003, Plasma interleukin-lbeta, -6, -8 and tumor necrosis factor-alpha as highly informative markers of pelvic inflammatory disease]; IL-2 or IL-8 (Interleukin-8) is related to Psoriasis [Iranian J Publ Health, Vol. 36, No. 2, 2007, pp. 87-91, Th1/Th2 Cytokines in Psoriasis (REVIEW); Clin Exp Immunol. 1995 Feb; 99 (2) : 148-54, IL-8/IL-8 receptor expression in psoriasis and the response to systemic tacrolimus (FK506) therapy]; IL-2 or IL-10 is related to graft versus host disease [Curr Pharm Des. 2004; 10 (11): 1195-205, Anti-cytokine therapy for the treatment of graft-versus-host disease. (REVIEW); Blood. 2002 Oct 1; 100 (7): 2650-8, Cytokine and chemokine profiles in autologous graft-versus-host disease (GVHD): interleukin 10 and interferon gamma may be critical mediators for the development of autologous GVHD] . From the above results, it can be seen that the inflammatory cytokines and the like are important therapeutic target molecules in the inflammatory disease, and if the secretion of the inflammatory cytokines is reduced, the inflammatory disease may be treated.

As used herein, the term "inflammatory disease" may be defined as any condition specified by a local or systemic bio-defensive response to infection of an external infectious source such as external physical and chemical irritation or bacteria, fungi, viruses, and various allergens. These reactions involve a series of complex physiological reactions such as activation of diverse inflammatory mediators and enzymes associated with immune cells (e.g., iNOS, COX-2, etc.), secretion of inflammatory mediators (e.g., NO, TNF-α, IL-6, IL-1β, PGE2 secretion), invasion of body fluids, cell migration, tissue destruction, etc., and may be manifested externally by symptoms including erythema, pain, edema, fever, deterioration or loss of specific functions of the body. The inflammatory disease may be acute, chronic, ulcerative, allergic or necrotic, and so far as any disease is included in the definition of such inflammatory diseases, whether the disease is acute, chronic, ulcerative, allergic or necrotic will not be an issue.

Specifically, in the present invention, the inflammatory diseases may include atopy, psoriasis, dermatitis, allergies, arthritis, rhinitis, otitis media, pharyngitis, tonsillitis, cystitis, nephritis, pelvicitis, inflammatory bowel disease, ankylosing spondylitis, systemic lupus erythematosus (SLE), atherosclerosis, asthma, arteriosclerosis, edema, rheumatoid arthritis, delayed allergy (type IV allergy), transplant rejection, graft versus host disease, autoimmune encephalomyelitis, multiple sclerosis, arthritis, cystic fibrosis, diabetic retinopathy, rhinitis, ischemic-reperfusion injury, vascular restenosis, glomerulonephritis, and gastrointestinal allergy, etc., but it is not limited thereto.

The anti-inflammatory composition of the present invention may also be provided as a pharmaceutical composition, food composition or cosmetic composition.

Further, the present invention may provide a method of preventing or treating an inflammatory disease, which includes administering an exosome containing glucosamine, a glucosamine derivative or a salt thereof, as an active ingredient to a subject suffering from the inflammatory disease.

In addition, the present invention may be to practice the use of a pharmaceutical composition for anti-inflammation.

According to another embodiment of the present invention, there is provided a composition for preventing, improving or treating intestinal diseases, which includes the exosome as an active ingredient.

In the present invention, the intestinal diseases may include diseases caused by a damage to the normal barrier function, such as inflammatory bowel disease (IBD), irritable bowel syndrome (IBS), traveler's diarrhea, constipation, acute diarrhea, enteritis, gastroenteritis, abdominal pain or abdominal distension.

The term "inflammatory bowel disease (IBD)" as used herein may include Crohn's disease, intestinal lesions involved in Behcet's disease, ulcerative colitis, hemorrhagic rectal ulcer and ileal cystitis, and refers to a group of diseases including Crohn's disease and ulcerative colitis. Ulcerative colitis only affects the large intestine. Inflammation and ulcers in ulcerative colitis are limited to the innermost two of the four layers in the large intestine, that is, the mucosal and submucosal layers. In Crohn's disease, inflammation and ulcers may extend through all layers of the barrier in both the small and large intestine.

As used herein, the term "irritable bowel syndrome (IBS)" is a chronic disease that accompanies abdominal pain and abdominal discomfort such as abdominal distension repeated for a long period of time without any organic cause, as well as a change in evacuation habit such as diarrhea, constipation, etc. Symptoms of this disease may be exacerbated due to any mental factor or stressful social environments. Depending on the predominant bowel movement disorder symptoms, IBS may include constipation, diarrhea or alternating IBS of diarrhea and constipation (also known as abbreviations IBS-C, IBS-D and IBS-A).

The exosome provided by the present invention may effectively prevent, improve or treat the above intestinal diseases by suppressing an expression of interleukin-2 (IL-2). It is already well known in the art to treat intestinal diseases if the expression of interleukin-2 is inhibited (Caprioli et al., J Clin Cell Immunol 2013, 4: 4).

The composition for preventing, improving or treating intestinal diseases of the present invention may also be provided in a form of a pharmaceutical composition or food composition.

Further, the present invention may provide a method of preventing or treating bowel disease, which includes administering the exosome containing glucosamine, a glucosamine derivative or a salt thereof, as an active ingredient to a subject having intestinal disease.

In addition, the present invention may be to practice the use of a pharmaceutical composition for prevention or treatment of intestinal diseases.

According to another embodiment of the present invention, there is provided a food composition for improving bowel function or promoting bowel movement, which includes the exosome provided by the present invention as an active ingredient.

In the present invention, the exosomes may prevent, improve or treat inflammatory bowel disease (IBD), irritable bowel syndrome (IBS), traveler's diarrhea, constipation, acute diarrhea, enteritis, gastroenteritis, abdominal pain or abdominal distension, thereby maintaining normal barrier function of the intestine, preventing, ameliorating or treating the damage to the intestine, and also facilitating normal bowel movement.

According to another embodiment of the present invention, there is provided a composition for improvement of skin, which includes the exosome provided by the present invention as an active ingredient.

As used herein, the term "skin improvement" refers to treatment, amelioration and/or relief of skin damage caused by intrinsic factors or exogenous factors or effects thereof, and more particularly, may include skin whitening, wrinkle improvement, elasticity enhancement, skin regeneration, skin moisturizing, anti-aging, alleviation of skin irritation, acne prevention or improvement, and/or atopic prevention or improvement effects, but it is not limited thereto.

Since the exosome containing glucosamine of the present invention has a very excellent ability of promoting stem cell proliferation, the skin condition may be improved by promoting or activating the proliferation of adult stem cells present in the skin when applying the exosome to the human body. The adult stem cells present in the skin may include, for example, stem cells present in a base layer of interfollicular epidermis between hair follicles, stem cells present in the hair follicle, or adipose-derived stems present in a subcutaneous fat layer.

The composition for skin improvement of the present invention may also be provided as a cosmetic composition, pharmaceutical composition or food composition.

Further, the exosome in the present invention has skin condition improving activity, and may be very useful as a composition for fillers for cosmetic or therapeutic purposes, more particularly, as a composition for filler injection. Specific examples thereof may include compositions for filling or replacement of bio-tissues, filling wrinkle, remodeling of the face or increasing lip volume, and rehydration of skin by mesotherapy.

According to another embodiment of the present invention, there is provided a composition for preventing, improving or treating a wound, which includes the exosome provided by the present invention as an active ingredient.

The composition according to the present invention has effects of promoting cell proliferation in keratinocytes, fibroblasts and skin stem cells, which have an important role in wound healing.

The composition for preventing, improving or treating wounds of the present invention may also be provided as a pharmaceutical composition, cosmetic composition or food composition.

Further, the present invention may provide a method for preventing or treating a wound, which includes administering the exosome containing glucosamine, a glucosamine derivative or a salt thereof, as an active ingredient to an injured subject.

Furthermore, the present invention may be to practice the use of a pharmaceutical composition for prevention or treatment of wounds.

According to another embodiment of the present invention, there is provided a composition for preventing, improving or treating hair loss, which includes the exosome provided by the present invention as an active ingredient.

As used herein, the term "hair loss" refers to a state in which hair is defective at a site where hair should normally be present, and generally means that the hair of the scalp falls out. Unlike virgin hair, which is thin hair without color, the grown hair may cause cosmetic problems when it is missing.

In the present invention, the hair loss may be clinically divided into two types involving scar formation and not, wherein one type of hair loss (scarring) in which the scar is formed does not regenerate the hair because the hair follicle is destroyed, whereas the other type of hair loss (not-scarring) retains hair follicles without formation of the scar, whereby the hair can regenerate after the symptom sites disappear.

In the present invention, the hair loss called alopecia may include alopecia areata, alopecia totalis, alopecia universalis, androgenetic alopecia, telogen effluvium, anagen effluvium, or chemotherapy-induced alopecia, but it is not limited thereto.

The exosome containing glucosamine of the present invention may significantly promote the proliferation of dermal papilla cells (DPC) and increase an expression level of hair growth or hair growth-related markers, thereby effectively preventing, improving or treating hair loss.

The composition for preventing, improving or treating hair loss of the present invention may also be provided as a pharmaceutical composition, cosmetic composition, or food composition.

Further, the present invention may provide a method for preventing or treating hair loss, which includes administering the exosome containing glucosamine, a glucosamine derivative or a salt thereof, as an active ingredient to a subject having hair loss.

Further, the present invention may be to practice the use of a pharmaceutical composition for prevention or treatment of hair loss.

As used herein, the term "prevention" may include any action that inhibits, suppresses or delays symptoms of various diseases using the composition of the present invention without limitation thereof.

In the present invention, "treatment" may include any symptom that improves or alleviates symptoms of various diseases using the composition of the present invention without limitation thereof.

In the present invention, the pharmaceutical composition may have a form of capsules, tablets, granules, injections, ointments, powders or beverages, and the pharmaceutical composition may be useful for humans.

The pharmaceutical composition of the present invention may be formulated and used in a form of oral formulations including, but it is not limited thereto, powders, granules, capsules, tablets, aqueous suspensions, as well as external preparations, suppositories, and sterile injection solutions, respectively, according to a conventional method. The pharmaceutical composition of the present invention may include a pharmaceutically acceptable carrier. Such pharmaceutically acceptable carriers may include: binders, lubricants, disintegrants, excipients, solubilizers, dispersants, stabilizers, suspending agents, pigments, flavoring agents, etc. for oral administration; buffers, preservatives, painless agents, solubilizers, isotonic agents, stabilizers, etc. which can be used in combination for injection; and bases, excipients, lubricants, preservatives, etc. for topical administration. The formulation of the pharmaceutical composition according to the present invention may be differently prepared by mixing the composition with any of the pharmaceutically acceptable carriers as described above. For example, for oral administration, the formulation may be prepared in a form of tablets, troches, capsules, elixirs, suspensions, syrups, wafers, etc. Further, in the case of injection, the formulation may be prepared in a form of unit dosage ampoules or multiple dosages. In addition, others such as solutions, suspensions, tablets, capsules and sustained release preparations may also be formulated.

Meanwhile, examples of carriers, excipients and diluents suitable for formulation may include lactose, dextrose, sucrose, sorbitol, mannitol, xylitol, erythritol, malditol, starch, acacia rubber, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate or mineral oil and the like. In addition, fillers, anti-coagulants, lubricants, wetting agents, fragrances, emulsifiers, preservatives, etc. may be further included.

A route of administration of the pharmaceutical composition according to the present invention may include, but it is not limited thereto, oral, intravenous, intramuscular, intraarterial, intramedullary, intrathecal, intracardiac, transdermal, subcutaneous, intraperitoneal, intranasal, intestinal, topical, sublingual or rectal administration. Herein, oral or parenteral administration is preferable.

In the present invention, "parenteral" includes subcutaneous, intradermal, intravenous, intramuscular, intra-articular, intra-synovial, intrasternal, epidural, intralesional and intracranial injection or infusion techniques. The pharmaceutical composition of the present invention may also be administered in a form of suppositories for rectal administration.

The pharmaceutical composition of the present invention may depend on a number of factors, including activity of specific compounds to be used, age, weight, general health, sex, diet, administration time, administration route, rate of discharge, drug combination, severity of a specific disease to be prevented or treated and the like. Further, the dosage of the pharmaceutical composition may vary depending on the patient's condition, weight, disease severity, dosage form, administration route and duration, however, may be appropriately selected by those skilled in the art. Specifically, a dose of 0.0001 to 50 mg/day/kg or 0.001 to 50 mg/day/kg may be administered. The administration may be done once a day, or divided into several times. The above dose is not defined to limit the scope of the present invention in any way. The pharmaceutical composition of the present invention may be formulated as pills, dragees, capsules, liquids, gels, syrups, slurries, or suspensions.

The cosmetic composition of the present invention may be formulated in a form of face lotion, nutrient lotion, nutrient essence, massage cream, beauty bath water additive, body lotion, body milk, bath oil, baby oil, baby powder, shower gel, shower cream, sunscreen lotion, sunscreen cream, suntan cream, skin lotion, skin cream, sunscreen cosmetics, cleansing milk, hair loss agent (for cosmetics), face and body lotion, face and body cream, skin whitening cream, hand lotion, hair lotion, cosmetic cream, jasmine oil, bath soap, water soap, beauty soap, shampoo, hand soap (hand cleaner), medicinal soap (non-medical use), cream soap, facial wash, whole body cleaner, scalp cleaner, hair rinse, cosmetic soap, tooth whitening gel, toothpaste, etc. To this end, the composition of the present invention may further include a solvent commonly used in the preparation of cosmetic composition, or a suitable carrier, excipient or diluent.

Types of the solvent further added to the cosmetic composition of the present invention may include, without particular limitation thereof, for example, water, saline, DMSO, or a combination thereof. Further, as the carrier, excipient or diluent, purified water, oil, wax, fatty acids, fatty acid alcohols, fatty acid esters, surfactants, humectants, thickeners, antioxidants, viscosity stabilizers, chelating agents, buffers, lower alcohols, and the like may be included, but it is not limited thereto. Furthermore, whitening agents, moisturizers, vitamins, sunscreens, perfumes, dyes, antibiotics, antibacterial agents, and antifungal agents may be included as necessary.

For the oil described above, hydrogenated vegetable oil, castor oil, cottonseed oil, olive oil, palm oil, jojoba oil, avocado oil may be used, while waxes used herein may include beeswax, mellow, carnauba, candelilla, montan, ceresin, liquid paraffin, and lanolin.

As the fatty acid, stearic acid, linoleic acid, linolenic acid and oleic acid may be used. Further, cetyl alcohol, octyl dodecanol, oleyl alcohol, panthenol, lanolin alcohol, stearyl alcohol, hexadecanol, etc. may be used as the fatty acid alcohol. As the fatty acid ester, isopropyl myristate, isopropyl palmitate and butyl stearate may be used. As the surfactant, cationic surfactants, anionic surfactants and nonionic surfactants known in the art may be used, while surfactants derived from natural products are preferably used.

In addition, the composition may include a desiccant, a thickener, an antioxidant, etc., which are widely known in the cosmetic field, and their types and amounts are adopted as known in the art.

The food composition of the present invention may be produced in a form of various foods, for example, beverages, gum, tea, vitamin complexes, powders, granules, tablets, capsules, cookies, rice cakes, breads and the like. Since the food composition of the present invention is composed of plant extracts having few toxicity and side effects, the composition may be safely used even for a long period of time for prevention purposes.

When the exosome of the present invention is included in the food composition, an amount thereof to be added may range from 0.1 to 50 wt.% based on a total weight of the composition.

In this regard, if the food composition is manufactured in a form of the beverage, there is no particular limitation other than the food composition contained in the weight ratio as indicated above. That is, like any commercially drinkable beverage, the beverage including the food composition of the present invention may contain any additional component such as a variety of flavors, natural carbohydrates, etc. In other words, as the natural carbohydrates, monosaccharides such as glucose, disaccharides such as fructose, sucrose, etc., common sugars such as polysaccharides, dextrins, cyclodextrins, and sugar alcohols such as xylitol, sorbitol, and erythritol may be included. The flavors may include, for examples, natural flavors (taumatine, stevia extracts (e.g., rebaudioside A, glycyrrhizine, etc.) and synthetic flavoring agents (saccharin, aspartame, etc.).

Other food compositions of the present invention may include diverse nutrients, vitamins, minerals (electrolytes), flavors such as synthetic flavors and natural flavors, coloring agents, pectic acid and salts thereof, alginic acid and salts thereof, organic acids, and protective colloidal thickeners, pH adjusters, stabilizers, preservatives, glycerin, alcohol, carbonic acid agent used in carbonated beverages and the like.

These ingredients may be used independently or in combination. A proportion of these additives is not so critical, but may be generally selected from 0.1 to about 50 parts by weight ("wt. parts") based on 100 wt. parts of the composition of the present invention.

According to another embodiment of the present invention, there is provided a screening method of a pharmaceutical composition or cosmetic composition including exosome, which contains glucosamine, a glucosamine derivative or a salt thereof, wherein the method includes: (1) separating the exosome from cells; and (2) measuring an expression level of the glucosamine, glucosamine derivative or salt thereof in the exosome.

The pharmaceutical composition may include a pharmaceutical composition for anti-inflammation, a pharmaceutical composition for prevention or treatment of intestinal diseases, a pharmaceutical composition for prevention or treatment of hair loss, a pharmaceutical composition for prevention or treatment of wounds, etc., but it is not limited thereto.

The cosmetic composition may be for skin improvement, but it is not limited thereto.

According to another embodiment of the present invention, there is provided a screening method of a pharmaceutical composition for anti-inflammation, including:
(1) separating the exosome from cells; and (2) measuring an expression level of the glucosamine, glucosamine derivative or salt thereof in the exosome.

In the present invention, the cells may be prokaryotic or eukaryotic cells. In the present invention, the prokaryotic cells may be bacterial cells, wherein the bacteria may be gram-negative or gram-positive bacteria. Further, in the present invention, the eukaryotic cells may include plant cells, animal cells or fungal cells.

According to one embodiment of the present invention, the cells described above may be derived from one or more microorganisms selected from the group consisting of, for example, Lactobacillus genus, Leuconostoc genus, Pediococcus genus, Lactococcus genus, Streptococcus genus, Aerococcus genus, Carnobacterium genus, Enterococcus genus, Oenococcus genus, Bifidobacterium genus, Sporolactobacillus genus, Tetragenococcus genus, Vagococcus genus, Weisella, Propionibacterium genus, Pediococcus genus, Staphylococcus genus, Peptostrepococcus genus, Bacillus genus, Micrococcus genus, Listeria genus, Escherichia genus, Debaromyces genus, Candida genus, Saccharomyces genus, Pichia genus, Torulopsis genus, Aspergillus genus, Rhizopus genus, Mucor genus, Penicillium genus, Bacteroides genus, Clostridium genus, Fusobacterium genus and Melissococcus genus.

According to one embodiment of the present invention, the cells may be derived from one or more microorganisms selected from the group consisting of, for example, Bacillus cereus, Bacillus licheniformis, Bacillus subtilis, Bifidobacterium bifidum, Bifidobacterium infantis, Bifidobacterium longum, Bifidobacterium lactis, Bifidobacterium animalis, Enterococcus Faecium, Enterococcus Faecalis, Lactobacillus acidopilus, Lactobacillus kefirgranum, Lactobacillus kefiranofaciens, Lactobacillus kefiri, Lactobacillus alimentarius, Lactobacillus bulgaricus, Lactobacillus casei, Lactobacillus curvatus, Lactobacillus delbrukii, Lactobacillus johnsonii, Lactobacillus farciminus, Lactobacillus gasseri, Lactobacillus helveticus, Lactobacillus rhamnosus, Lactobacillus reuteri, Lactobacillus sakei, Lactococcus lactis, Lactobacillus brevis, Lactobacillus plantarum, Lactobacillus pentosus, Lactobacillus kimchi, Lactobacillus confusus, Lactobacillus curvatus, Streptococcus faecium, Streptococcus faecalis, Streptococcus agalactiae, Streptococcus mutans, Streptococcus thermophilus, Streptococcus lactis, Weissella confusa, Weissella koreensis, Weissella kimchii, Weissella cibaria, Weissella viridescens, Pediococcus acidilactici, Pediococcus pentosaceus, Pediococcus acidilactici, Pediococcus halophilus, Staphylococcus carnosus, Staphylococcus xylosus, Staphylococcus aureus, Escherichia coli, Tetragenococcus halophilus, Saccharomyces cerevisiae, Saccharomyces servazzii, Saccharomyces boulardii and Listeria monocytogens, but it is not limited thereto.

In the present invention, the method of measuring the expression level of the glucosamine, glucosamine derivative or salt thereof is not particularly limited, and may include, for example, chromatography (e.g., high performance liquid chromatography, thin layer chromatography (TLC)), mass spectrometry, hexosamine assay, etc. but it is not limited thereto.

In the present invention, if the glucosamine, glucosamine derivative or salt thereof is present in the exosome isolated as above or if the glucosamine, glucosamine derivative or salt thereof is included in the exosome in an amount of: more than 0 and 20 wt.% or less, more than 0 and 19 wt.% or less, more than 0 and 18 wt.% or less, more than 0 and 17 wt.% or less, more than 0 and 16 wt.% or less, more than 0 and 15 wt.% or less, more than 0 and 14 wt.% or less, more than 0 and 13 wt.% or less, more than 0 and 12 wt.% or less, more than 0 and 11 wt.% or less, more than 0 and 10 wt.% or less, more than 0 and 9 wt.% or less, more than 0 and 8 wt.% or less, more than 0 and 7 wt.% or less, more than 0 and 6 wt.% or less, more than 0 and 5 wt.% or less, 0.1 to 10 wt.%, 0.1 to 9 wt. %, 0.1 to 8 wt.%, 0.1 to 7 wt.%, 0.1 to 6 wt.%, or 0.1 to 5 wt.%, a process of determining the exosome as an anti-inflammatory agent for prevention, improvement or treatment of inflammatory diseases may also be included.

In the present invention, the inflammatory diseases may include atopy, psoriasis, dermatitis, allergies, arthritis, rhinitis, otitis media, pharyngitis, tonsillitis, cystitis, nephritis, pelvicitis, inflammatory bowel disease, ankylosing spondylitis, systemic lupus erythematosus (SLE), atherosclerosis, asthma, arteriosclerosis, edema, rheumatoid arthritis, delayed allergy (type IV allergy), transplant rejection, graft versus host disease, autoimmune encephalomyelitis, multiple sclerosis, arthritis, cystic fibrosis, diabetic retinopathy, rhinitis, ischemic-reperfusion injury, vascular restenosis, glomerulonephritis, and gastrointestinal allergy, etc., but it is not limited thereto.

According to another embodiment of the present invention, there is provided a screening method of agents for prevention or treatment of intestinal diseases, which includes: (1) separating the exosome from cells; and
(2) measuring an expression level of the glucosamine, glucosamine derivative or salt thereof in the exosome.

In the present invention, definition of the cells and the method of measuring the expression level of the glucosamine, glucosamine derivative or salt thereof substantially overlap with those described in the screening method of the anti-inflammatory agent, and therefore will not be described in detail below.

In the present invention, the glucosamine, glucosamine derivative or salt thereof is present in the exosome isolated as described above or if the glucosamine, glucosamine derivative or salt thereof is included in the exosome in an amount of: more than 0 and 20 wt.% or less, more than 0 and 19 wt.% or less, more than 0 and 18 wt.% or less, more than 0 and 17 wt.% or less, more than 0 and 16 wt.% or less, more than 0 and 15 wt.% or less, more than 0 and 14 wt.% or less, more than 0 and 13 wt.% or less, more than 0 and 12 wt.% or less, more than 0 and 11 wt.% or less, more than 0 and 10 wt.% or less, more than 0 and 9 wt.% or less, more than 0 and 8 wt.% or less, more than 0 and 7 wt.% or less, more than 0 and 6 wt.% or less, more than 0 and 5 wt.% or less, 0.1 to 10 wt.%, 0.1 to 9 wt.%, 0.1 to 8 wt.%, 0.1 to 7 wt.%, 0.1 to 6 wt.%, or 0.1 to 5 wt.%, a process of determining the exosome as an agent for prevention or treatment of intestinal diseases may also be included.

In the present invention, the intestinal diseases may include diseases caused by a damage to the normal barrier function, such as inflammatory bowel disease (IBD), irritable bowel syndrome (IBS), traveler's diarrhea, constipation, acute diarrhea, enteritis, gastroenteritis, abdominal pain or abdominal distension. Herein, the inflammatory bowel disease (IBD) may include Crohn's disease, intestinal lesions involved in Behcet's disease, ulcerative colitis, hemorrhagic rectal ulcer or ileal cystitis, while the irritable bowel syndrome (IBS) may include constipation, diarrhea or alternating IBS of diarrhea and constipation (also known as abbreviations IBS-C, IBS-D and IBS-A).

According to another embodiment of the present invention, there is provided a screening method of an agent for skin improvement, which includes: (1) separating the exosome from cells; and
(2) measuring an expression level of the glucosamine, glucosamine derivative or salt thereof in the exosome.

In the present invention, definition of the cells and the method of measuring the expression level of the glucosamine, glucosamine derivative or salt thereof substantially overlap with those described in the screening method of the anti-inflammatory agent, and therefore will not be described in detail below.

In the present invention, if the glucosamine, glucosamine derivative or salt thereof is present in the exosome isolated as described above or if the glucosamine, glucosamine derivative or salt thereof is included in the exosome in an amount of: more than 0 and 20 wt.% or less, more than 0 and 19 wt.% or less, more than 0 and 18 wt.% or less, more than 0 and 17 wt.% or less, more than 0 and 16 wt.% or less, more than 0 and 15 wt.% or less, more than 0 and 14 wt.% or less, more than 0 and 13 wt.% or less, more than 0 and 12 wt.% or less, more than 0 and 11 wt.% or less, more than 0 and 10 wt.% or less, more than 0 and 9 wt.% or less, more than 0 and 8 wt.% or less, more than 0 and 7 wt.% or less, more than 0 and 6 wt.% or less, more than 0 and 5 wt.% or less, 0.1 to 10 wt.%, 0.1 to 9 wt.%, 0.1 to 8 wt.%, 0.1 to 7 wt.%, 0.1 to 6 wt.%, or 0.1 to 5 wt.%, a process of determining the exosome as an agent for skin improvement may also be included.

According to another embodiment of the present invention, there is provided a screening method of a wound treatment agent, which includes: (1) separating the exosome from cells; and
(2) measuring an expression level of the glucosamine, glucosamine derivative or salt thereof in the exosome.

In the present invention, definition of the cells and the method of measuring the expression level of the glucosamine, glucosamine derivative or salt thereof substantially overlap with those described in the screening method of the anti-inflammatory agent, and therefore will not be described in detail below.

In the present invention, if the glucosamine, glucosamine derivative or salt thereof is present in the exosome isolated as described above or if the glucosamine, glucosamine derivative or salt thereof is included in the exosome in an amount of: more than 0 and 20 wt.% or less, more than 0 and 19 wt.% or less, more than 0 and 18 wt.% or less, more than 0 and 17 wt.% or less, more than 0 and 16 wt.% or less, more than 0 and 15 wt.% or less, more than 0 and 14 wt.% or less, more than 0 and 13 wt.% or less, more than 0 and 12 wt.% or less, more than 0 and 11 wt.% or less, more than 0 and 10 wt.% or less, more than 0 and 9 wt.% or less, more than 0 and 8 wt.% or less, more than 0 and 7 wt.% or less, more than 0 and 6 wt.% or less, more than 0 and 5 wt.% or less, 0.1 to 10 wt.%, 0.1 to 9 wt.%, 0.1 to 8 wt.%, 0.1 to 7 wt.%, 0.1 to 6 wt.%, or 0.1 to 5 wt.%, a process of determining the exosome as a wound treatment agent may also be included.

According to another embodiment of the present invention, there is provided a screening method of an agent for prevention or treatment of hair loss, which includes: (1) separating the exosome from cells; and
(2) measuring an expression level of the glucosamine, glucosamine derivative or salt thereof in the exosome.

In the present invention, definition of the cells and the method of measuring the expression level of the glucosamine, glucosamine derivative or salt thereof substantially overlap with those described in the screening method of the anti-inflammatory agent, and therefore will not be described in detail below.

In the present invention, if the glucosamine, glucosamine derivative or salt thereof is present in the exosome isolated as described above or if the glucosamine, glucosamine derivative or salt thereof is included in the exosome in an amount of: more than 0 and 20 wt.% or less, more than 0 and 19 wt.% or less, more than 0 and 18 wt.% or less, more than 0 and 17 wt.% or less, more than 0 and 16 wt.% or less, more than 0 and 15 wt.% or less, more than 0 and 14 wt.% or less, more than 0 and 13 wt.% or less, more than 0 and 12 wt.% or less, more than 0 and 11 wt.% or less, more than 0 and 10 wt.% or less, more than 0 and 9 wt.% or less, more than 0 and 8 wt.% or less, more than 0 and 7 wt.% or less, more than 0 and 6 wt.% or less, more than 0 and 5 wt.% or less, 0.1 to 10 wt.%, 0.1 to 9 wt.%, 0.1 to 8 wt.%, 0.1 to 7 wt.%, 0.1 to 6 wt.%, or 0.1 to 5 wt.%, a process of determining the exosome as a wound treatment agent may also be included.

### [Advantageous Effects]

The exosome including glucosamine, a glucosamine derivative or a salt thereof provided by the present invention have excellent anti-inflammatory effects, thereby effectively preventing, improving or treating a variety of inflammatory diseases. In addition, the exosome described above also exhibit excellent therapeutic effects in treatment of intestinal diseases, skin improvement, or treatment of wound or hair loss.

### [Brief Description of Drawings]

FIG. 1 is a graph illustrating results of measuring a change in the expression level of inflammatory cytokine (IL-2) as compared to the control, after treatment of Jurkat cells in Experimental Example 1 with the exosome containing glucosamine isolated from each microorganism strain.
FIG. 2 is a graph illustrating results of measuring a change in the expression level of inflammatory cytokine (IL-2), as compared to the control, after treatment of Jurkat cells in Experimental Example 2 with a Lactobacillus kefirgranum conditioned medium, the exosome containing glucosamine separated from the conditioned medium or the conditioned medium from which the exosome was removed, respectively.
FIG. 3 is a graph illustrating results of measuring a change in the expression level of inflammatory cytokines (IL-2) as compared to the control, after treatment of Jurkat cells in Experimental Example 3 with the exosome containing glucosamine, liposome containing glucosamine or glucosamine alone, respectively.
FIGS. 4A to 4D illustrate cross-sectional photographs following H & E staining of colon tissues of mice after treatment of a colitis mouse model in Experimental Example 4 with the exosome containing glucosamine.
FIG. 5 is a graph illustrating scores evaluated by observing a length and appearance of the large intestine, after treatment of the colitis mouse model in Experimental Example 4 with the exosome containing glucosamine.
FIG. 6 is a graph illustrating results of evaluating homeostasis of feces, after treatment of the colitis mouse model in Experimental Example 4 with the exosome containing glucosamine.
FIG. 7 is a graph illustrating results of evaluating a degree of bleeding in feces, after treatment of the colitis mouse model in Experimental Example 4 with the exosome containing glucosamine.
FIG. 8 is a graph illustrating results of measuring a change in the body weight of mice, after treatment of the colitis mouse model in Experimental Example 4 with the exosome containing glucosamine.
FIG. 9 is a graph illustrating results of measuring a change in the cell proliferation rate, after treatment of the keratinocytes (HaCaT) in Experimental Example 5 with the exosome containing glucosamine by concentration.
FIG. 10 is a graph illustrating results of measuring a change in the cell proliferation rate, after treatment of skin fibroblasts (HDF) in Experimental Example 5 with the exosome containing glucosamine by concentration.
FIG. 11 is a graph illustrating results of measuring a change in the cell proliferation rate, after treatment of the adipose-derived stem cells (ASC) in Experimental Example 6 with the exosome containing glucosamine.

### [Mode for Carrying out Invention]

The present invention relates to an exosome including glucosamine, a glucosamine derivative or a salt thereof.

Hereinafter, the present invention will be described in more detail by means of the following examples. These examples are proposed only for describing the present invention in more detail, and it will be apparent to those skilled in the art that the scope of the present invention is not limited by these examples according to the gist of the present invention.

### EXAMPLE

### [Example 1] Confirmation of glucosamine in exosome

### 1. Isolation of exosome

Lactobacillus kefirgranum, Lactobacillus kefiranofaciens, Lactobacillus kefiri, Weissella koreensis, Tetragenococcus halophilus and Bifidobacterium animalis strains were inoculated in MRS medium (including 10 g of proteose peptone, 10 g of beef extract, 5 g of yeast extract, 20 g of D-glucose, 1 ml of Tween 80, 2 g of K₂HPO₄, 5 g of sodium acetate, 2 g of diammonium hydrogencitrate, 0.2 g of MgSO₄·7H₂O, 0.2 g of MnSO₄·H₂O and 1 L of distilled water, pH 6.2-6.5), followed by stationary incubation at 30 °C and 0 rpm. Further, Staphylococcus aureus, Listeria monocytogens and Escherichia coli were inoculated in LB medium (including 10 g of tryptone, 5 g of yeast extract, 5 g of NaCl and 1 L of distilled water, pH 6.8-7.2), followed by stationary incubation at 30 °C and 0 rpm.

The culture medium thus obtained for each strain was centrifuged at 300 g and 4 °C for 10 minutes, the supernatant was centrifuged at 1,200 g and 4 °C for 20 minutes, and then the supernatant was taken again at 10,000 g and 4 °C for 30 minutes. After centrifugation, the supernatant was taken and centrifuged at 110,000 g and 4 °C for 1 hour and 10 minutes through an ultracentrifuge, and then the supernatant was removed to suspend the precipitate with PBS to obtain exosome.

### 2. Measurement of glucosamine content

As described above, a content of glucosamine in the exosome separated from each strain was measured by hexosamine assay. Specifically, the content of hexamine was measured by a 3-methyl-2-benzothia-zolinone hydrazone hydrochloride (MBTH) method (Frederik et al., 2000). That is, 100 µl of 1M HCl solution was added to a cap tube containing 100 µl of the sample, heated in a Reacti-thermometer at 110 °C for 2 hours, then cooled, and 400 µl of 2.5% sodium nitrate was added thereto, followed by leaving the same at room temperature for 15 minutes. Then, 200 µl of 4M ammonium sulfamate was added to the mixed solution, left at room temperature for 5 minutes, and 200 µl of 0.25% MBTH was further added and incubated in a water bath at 37 °C for 30 minutes. Thereafter, 200 µl of 1.0% ferric chloride was added and incubated in a water bath at 37 °C for 5 minutes, followed by cooling the same to measure absorbance at 650 nm. A content of hexamine was determined according to the standard curve prepared with D-glucosamine, and results of the content of glucosamine in each exosome are shown in Table 1 below.

**[TABLE 1]**

| Item | | Content of glucosamine (wt.%) |
|---|---|---|
| P1 | Control | - |
| P2 | Lactobacillus kefirgranum | 2.61 |
| P3 | Lactobacillus kefiranofaciens | 2.50 |
| P4 | Lactobacillus kefiri | 2.76 |
| P5 | Staphylococcus aureus | 1.85 |
| P6 | Listeria monocytogens | 0.75 |
| P7 | Escherichia coli | Substantially not detected |
| P8 | Weissella koreensis | Substantially not detected |
| P9 | Tetragenococcus halophilus | Substantially not detected |
| P10 | Bifidobacterium animalis | 2.74 |
| P11 | Cyclosporin A | - |

As shown in Table 1, it was determined that glucosamine was present in the exosomes of P2 to P6 and P10, respectively, but glucosamine was not substantially detected in the exosomes of P7 to P9.

### [Experimental Example 1] Confirmation of anti-inflammatory effect of exosome containing glucosamine (1)

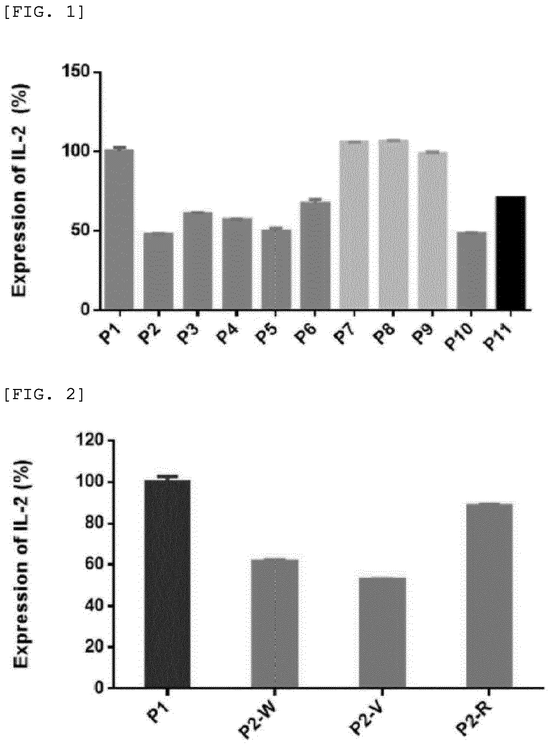

After inoculating Jurkat cells with 4 x 10⁴ cells/well in each well of a 96-well plate, each exosome prepared in Example 1 was used in an amount of 100 µl/well to treat the cells, and cultured for 24 hours. Thereafter, an expression level of cytokine IL-2 related to proliferation and differentiation of T cells acting as a cause of inflammatory bowel disease was measured by ELISA, and a change in the expression level of IL-2 compared to the untreated control was determined. Results thereof are shown in Table 2 below and FIG. 1. However, as a positive control (P11), cyclosporin A, which is used as a therapeutic agent for inflammatory bowel disease, was used in the same amount as the exosome to treat the cells.

**[TABLE 2]**

| Item | IL-2 (%) |
|---|---|
| P1 | 100 |
| P2 | 48 |
| P3 | 61 |
| P4 | 57 |
| P5 | 50 |
| P6 | 67 |
| P7 | 106 |
| P8 | 106 |
| P9 | 98 |
| P10 | 48 |
| P11 | 71 |

As shown in Table 2 above and FIG. 1, it was confirmed that IL-2 expression level was significantly reduced when treated with the exosomes (P2 to P6 and P10) containing glucosamine according to the present invention, and it was confirmed that the exosome of the present invention has superior results over cyclosporin A used as a typical therapeutic agent for inflammatory bowel disease.

As such, it can be seen that the exosome containing glucosamine according to the present invention may be used as an anti-inflammatory agent.

### [Experimental Example 2] Confirmation of anti-inflammatory effect of exosome containing glucosamine (2)

In order to demonstrate whether the anti-inflammatory effect of Experimental Example 1 is based on effects of the exosome containing glucosamine only, the culture medium (P2-W) of Lactobacillus kefirgranum in Example 1, the exosome (P2-V) containing glucosamine separated from the culture medium, and the culture medium (P2-R) from which the exosome was removed, respectively, were used to treat Jurkat cells in the same manner as in Experimental Example 1, followed by measuring the expression level of IL-2. Results thereof are shown in Table 3 below and FIG. 2.

**[TABLE 3]**

| Item | | IL-2 (%) |
|---|---|---|
| P1 | Control | 100 |
| P2-W | Lactobacillus kefirgranum conditioned medium | 62 |
| P2-V | Exosome containing Lactobacillus kefirgranum-derived glucosamine | 53 |
| P2-R | Lactobacillus kefirgranum conditioned medium with exosome removed | 88 |

As shown in Table 3 above and FIG. 2, the IL expression level was significantly reduced when the exosome containing glucosamine (P2-V) or the culture medium (P2-W) including the same was used for treatment according to the present invention. In particular, for the exosome containing glucosamine (P2-V), it was found that a degree of reduction in the IL-2 expression level was superior over that of the culture medium. However, it can be confirmed that IL-2 expression reduction effects are insignificant when treated with the culture medium (P2-R) from which the exosome was removed.

As such, it can be seen that the exosome containing glucosamine according to the present invention may be used as an anti-inflammatory agent.

### [Experimental Example 3] Confirmation of anti-inflammatory effect of exosomes containing glucosamine (3)

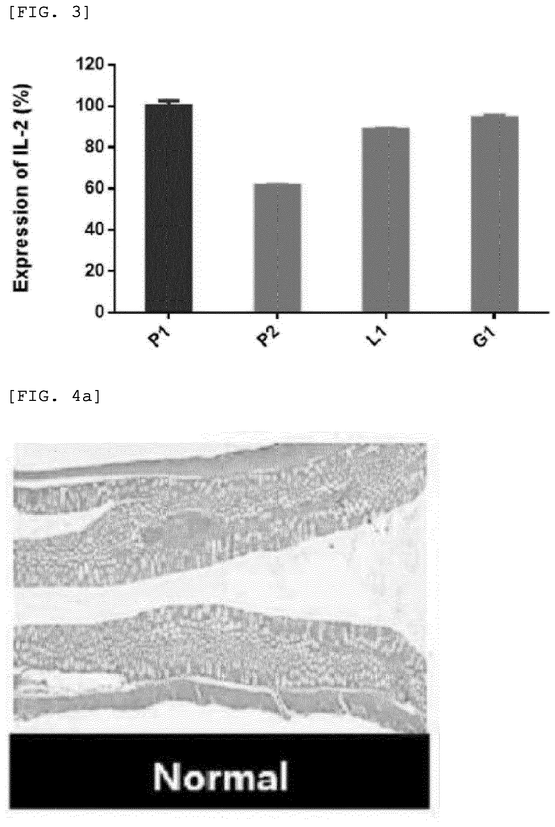

In order to demonstrate whether the anti-inflammatory effects of Experimental Examples 1 and 2 are not due to glucosamine alone but to the effect of the exosome containing glucosamine, the exosome containing glucosamine, which was separated from the culture medium of Lactobacillus kefirgranum in Example 1, liposome containing glucosamine (Lipofectamine 2000) and glucosamine itself, respectively, were used at a dose of 4 µg/ml to treat Jurkat cells as in Experimental Example 1 above, followed by measuring the IL-2 expression level. Results thereof are shown in Table 4 below and FIG. 3.

**[TABLE 4]**

| Item | | IL-2 (%) |
|---|---|---|
| P1 | Control | 100 |
| P2 | Exosome containing Lactobacillus kefirgranum-derived glucosamine | 62 |
| L1 | Liposome containing glucosamine | 89 |
| G1 | Glucosamine | 94 |

As shown in Table 4 above and FIG. 3, when the exosome (P2) containing glucosamine was used for treatment according to the present invention, the IL-2 expression level was significantly reduced. However, when the liposome (L1) containing glucosamine or glucosamine itself (G1) was directly used for treatment, it could be confirmed that there was almost no effect of reducing IL-2 expression.

As such, it can be seen that the exosome containing glucosamine according to the present invention may be used as an anti-inflammatory agent.

### [Experimental Example 4] Confirmation of therapeutic effects of glucosamine-containing exosome against intestinal disease

In BALB/c mice, colitis was induced with 2,4,6-trinitrobenzenesulfonic acid (TNBS). Specifically, after anesthetizing each mouse with ether, a solution in which 2.5 g of 2,4,6-trinitrobenzene sulfonic acid (TNBS) is mixed with 50% ethanol was administered into the large intestine through the anus using a syringe of 1 ml dose having a round end by 0.1 ml, followed by holding the mouse vertically for 30 seconds to cause inflammation. On the other hand, 0.1 ml of physiological saline was orally administered to the normal group. Thereafter, the exosome (P2) containing glucosamine obtained in Example 1 was administered orally at a dose of 600 pg/mouse (30 mg/kg) once a day for 10 days from the next day. After the mouse was subjected to euthanasia with suffocation using carbon dioxide the day after sample administration was completed, the large intestine was cut between the cecum and the site immediately before the anus. On the other hand, as a positive control, prednisolone (P12) as a therapeutic agent for colitis was administered orally in an amount of 2 mg/kg.

### (1) Tissue appearance assessment

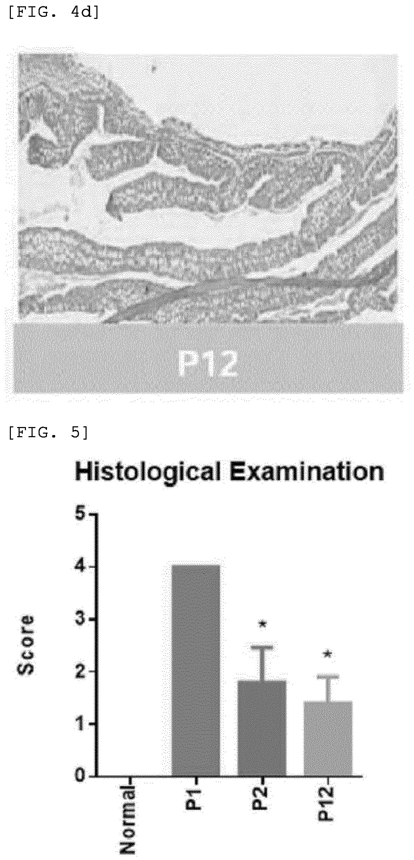

Cross-sectional photographs, which are taken after H & E staining of the extracted large intestine tissue, are shown in FIGS. 4A to 4D. In addition, length and appearance of the extracted bowel tissue were observed to score according to the criteria stated in Table 5 below (Hollenbach et al., 2005 criteria for colitis). Results thereof are shown in FIG. 5.

**[TABLE 5]**

| Macroscopic score | Standard |
|---|---|
| 0 | No ulcers and inflammation found |
| 1 | Redness without bleeding found |
| 2 | Congestive ulcers found |
| 3 | Ulcer and inflammation found in only one site |
| 4 | Ulcers and inflammation found in two or more sites |
| 5 | Ulcer enlarged to 2 cm or more |

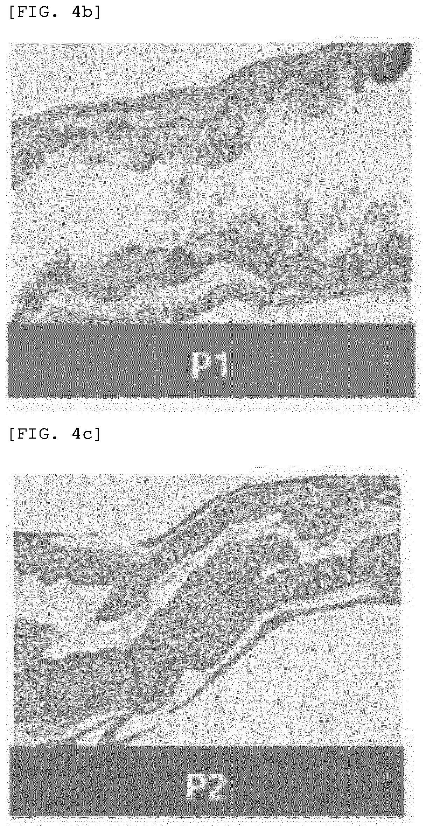

As shown in FIG. 4B, severe epithelial necrosis, bleeding, inflammatory cell infiltration, edema and ulcers were observed in the large intestine of the colitis mouse model. However, in the case of administering the exosome (FIG. 4C, P2) containing glucosamine according to the present invention, it could be confirmed that symptoms of colitis were significantly alleviated at a level equal to or higher than the case of administering the prednisolone (FIG. 4D, P12) .

Further, as shown in FIG. 5, when the exosome (P2) containing glucosamine according to the present invention was administered to the colitis mouse model, bleeding in the bowel was reduced and it was confirmed that such therapeutic effects are substantially equal to the case of administering the positive control, that is, the prednisolone in a large amount of 2 mg/kg.

### (2) DAI evaluation

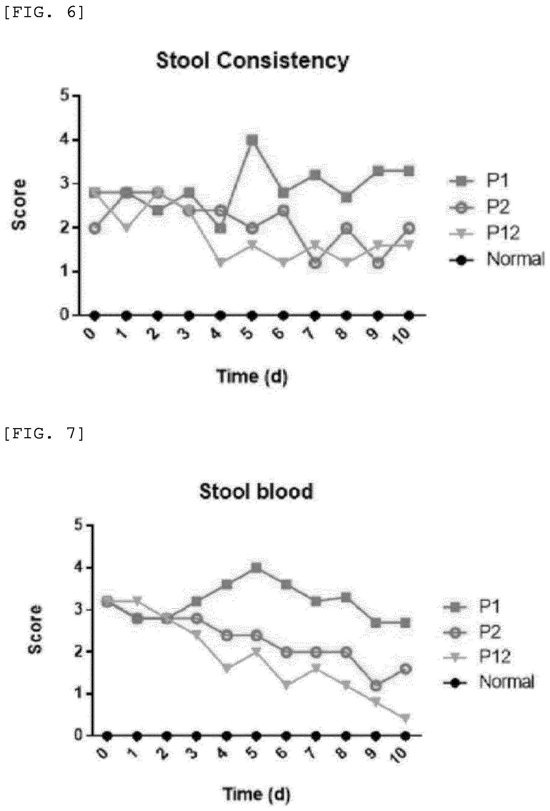

DAI (disease activity index) is a method for scoring symptoms due to TNBS causing acute colitis, which begins at a time of providing the exosome of the present invention, in order to measure a concentration and check color of the stool at the same time every day during scoring. Stool consistency, stool bleeding and the weight of the mouse were measured, and the measured results are shown in FIGS. 6 to 8, respectively. In this case, the evaluation criteria of DAI are shown in Table 6 below.

**[TABLE 6]**

| Stool consistency | | Stool bleeding | |
|---|---|---|---|
| 0 | Formed | 0 | Normal color |
| 2 | Loose stool | 2 | Fecal occult blood test positive |
| 4 | Diarrhea | 4 | Gross bleeding |

As shown in FIGS. 6 to 8, when the exosome (P2) containing glucosamine according to the present invention was administered to the colitis mouse model, it could be confirmed that the DAI value was lowered to a level equal to or higher than that of the prednisolone (P12) as a positive control, thereby improving the bowel movement, while not exhibiting a significant change in the body weight of the mouse.

As such, it can be seen that the exosome containing glucosamine according to the present invention may be used as a therapeutic agent for intestinal diseases.

### [Experimental Example 5] Effect of promoting proliferation of skin cells by exosomes containing glucosamine

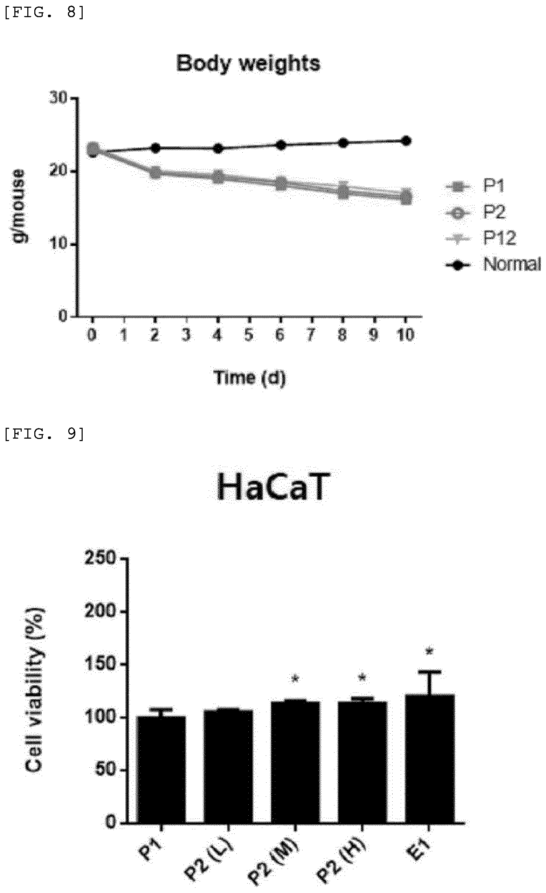

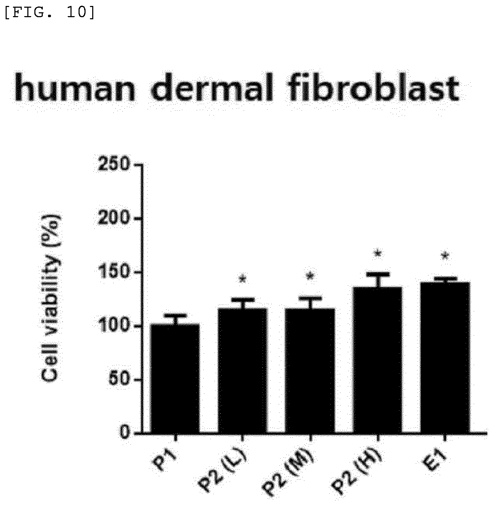

After inoculating 3,000 keratinocytes (human keratinocyte cell line, HaCaT) and 3,000 human fibroblasts (HDF) into DMEM medium containing 1% FBS, as shown in Table 7 below, the cell line was treated with the exosome of the present invention, EGF or FBS. After 48 hours, a change in cell viability was measured, and results thereof are shown in Tables 8 and 9 below and FIGS. 9 and 10.

**[TABLE 7]**

| | | |
|---|---|---|
| Item | Treatment group | Concentration |
| P1 | Negative control | - |
| P2 (L) | P2 exosome | 1 x 10^8/ml |
| P2 (M) | P2 exosome | 1 x 10^9/ml |
| P2 (H) | P2 exosome | 1 x 10^10/ml |
| E1 | EGF | 500 ng/ml |

**[TABLE 8]**

| Item | HaCaT proliferation rate (%) |
|---|---|
| P1 | 93.84 |
| P2 (L) | 107.25 |
| P2 (M) | 114.20 |
| P2 (H) | 116.68 |
| E1 | 160.38 |

**[TABLE 9]**

| Item | HDF proliferation rate (%) |
|---|---|
| P1 | 97.18 |
| P2 (L) | 101.39 |
| P2 (M) | 120.95 |
| P2 (H) | 134.62 |
| E1 | 143.04 |

As shown in Tables 8 and 9 above and FIGS. 9 and 10, when the exosome according to the present invention was used to treat keratinocytes (HaCaT) and skin fibroblasts (HDF), it could be seen that the cell proliferation rate was significantly increased depending on the concentration.

### [Experimental Example 6] Effect of promoting proliferation of stem cells by exosome containing glucosamine

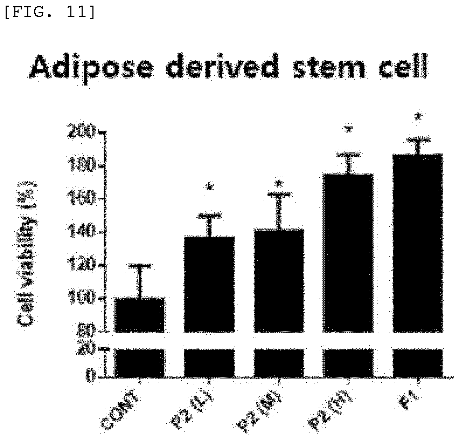

Adipocytes derived stem cells (ADCs) were cultured in DMEM medium (Invitrogen) containing 10% fetal calf serum and 1% penicillin/streptomycin at 37 °C and 5% CO₂ conditions, and the medium was changed every 3 days. After culturing for 24 hours, the cells were treated under the conditions shown in Table 10 below, and further cultured for 24 hours. After removing the treated material from each cell and dispensing the CCK-8 solution thereto, the cells were incubated at 37 °C in a CO₂ incubator for 2 hours, and absorbance was measured at 450 nm. Results thereof are shown in Table 11 below and FIG. 11.

**[TABLE 10]**

| Item | Treatment group | Concentration |
|---|---|---|
| CONT | Negative control | - |
| P2 (L) | P2 exosome | 1 x 10^(n-1)/ml |
| P2 (M) | P2 exosome | 1 x 10^(n)/ml |
| P2 (H) | P2 exosome | 1 x 10^(n+1)/ml |
| F1 | FBS | 10 wt.% in medium |

**[TABLE 11]**

| Item | ASC proliferation rate (%) |
|---|---|
| P1 | 99.61 |
| P2 (L) | 136.54 |
| P2 (M) | 140.91 |
| P2 (H) | 174.44 |
| E1 | 186.10 |

As shown in Table 11 above and FIG. 11, when adipose-derived stem cells (ASC) were treated with the exosome according to the present invention, it could be confirmed that the cell proliferation rate was significantly increased depending on the concentration, and the degree of increase was substantially equal to the level when treated with fetal bovine serum (FBS).

### [Experimental Example 7] Effect of promoting proliferation of dermal papilla cells by exosome containing glucosamine

After dispensing a serum medium with 10,000 cells/well of papillary cells (Dermal papilla cells, DPC) in a 96-well microplate, the cells were incubated overnight at 37 °C in a CO₂ incubator. After incubation, the papillary cells were treated as shown in Table 12 below, and further cultured for 24 hours. After removing the treated material from each cell and dispensing the CCK-8 solution thereto, the cells were incubated in the CO₂ incubator at 37 °C for 2 hours, and absorbance was measured at 450 nm. The measured results are shown in Table 13 below.

**[TABLE 12]**

| Item | Treatment group | Concentration |
|---|---|---|
| P1 | Negative control | - |
| P2 (M) | P2 exosome | 1 x 10^(n)/ml |
| P2 (H) | P2 exosome | 1 x 10^(n+1)/ml |
| M1 | Minoxidil | 10 µM |

**[TABLE 13]**

| Item | DPC proliferation rate (%) |
|---|---|
| P1 | 97.98 |
| P2 (M) | 108.86 |
| P2 (H) | 126.75 |
| M1 | 118.97 |

As shown in Table 13 above, when the exosome according to the present invention was used to treat dermal papilla cells (DPC), it could be confirmed that the cell proliferation rate was significantly increased depending on the concentration, and the degree of increase was higher than the case of treatment using minoxidil, which is commonly used as a therapeutic agent for hair growth.

As such, it can be seen that the exosome containing glucosamine according to the present invention may be used as a therapeutic agent for hair loss.

### [Industrial Applicability]

The present invention provides novel exosomes and various uses thereof.

The present invention relates to a novel exosome comprising glucosamine, glucosamine derivatives, or salts thereof. The exosome provided by the present invention has an excellent anti-inflammatory effect, and thus can effectively prevent, ameliorate, or treat various inflammatory diseases. The exosome also has an excellent effect of treating intestinal diseases, improving the skin, treating wounds, or treating hair loss.

## Claims

1. An exosome comprising glucosamine, a glucosamine derivative or a salt thereof.

2. The exosome according to claim 1, wherein the exosome is derived from a prokaryotic cell or eukaryotic cell.

3. The exosome according to claim 1, wherein the exosome is derived from one or more microorganisms selected from the group consisting of Lactobacillus genus, Leuconostoc genus, Pediococcus genus, Lactococcus genus, Streptococcus genus, Aerococcus genus, Carnobacterium genus, Enterococcus genus, Oenococcus genus, Bifidobacterium genus, Sporolactobacillus genus, Tetragenococcus genus, Vagococcus genus, Weisella, Propionibacterium genus, Pediococcus genus, Staphylococcus genus, Peptostrepococcus genus, Bacillus genus, Micrococcus genus, Listeria genus, Escherichia genus, Debaromyces genus, Candida genus, Pichia genus, Torulopsis genus, Aspergillus genus, Rhizopus genus, Mucor genus, Penicillium genus, Bacteroides genus, Clostridium genus, Fusobacterium genus and Melissococcus genus.

4. The exosome according to claim 1, wherein the glucosamine derivative is **characterized in that** hydrogen of at least one hydroxyl group in glucosamine is substituted with an acyl or alkyl group.

5. The exosome according to claim 1, wherein the glucosamine, glucosamine derivative or salt thereof is included in an amount of more than 0 and 20% by weight ("wt. %") or less.

6. A cell culture, comprising an exosome which includes glucosamine, a glucosamine derivative or a salt thereof.

7. A cell culture medium, comprising an exosome which includes glucosamine, a glucosamine derivative or a salt thereof.

8. A composition for promoting proliferation of stem cells, comprising an exosome which includes glucosamine, a glucosamine derivative or a salt thereof.

9. A medium composition for culturing stem cells, comprising an exosome which includes glucosamine, a glucosamine derivative or a salt thereof.

10. A pharmaceutical composition for anti-inflammation, comprising an exosome which includes glucosamine, a glucosamine derivative or a salt thereof.

11. The pharmaceutical composition for anti-inflammation according to claim 10, wherein the composition is used to prevent or treat inflammatory diseases.

12. The pharmaceutical composition for anti-inflammation according to claim 11, wherein the inflammatory diseases are any one of atopy, psoriasis, dermatitis, allergies, arthritis, rhinitis, otitis media, pharyngitis, tonsillitis, cystitis, nephritis, pelvicitis, inflammatory bowel disease, ankylosing spondylitis, systemic lupus erythematosus (SLE), atherosclerosis, asthma, arteriosclerosis, edema, rheumatoid arthritis, delayed allergy (type IV allergy), transplant rejection, graft versus host disease, autoimmune encephalomyelitis, multiple sclerosis, arthritis, cystic fibrosis, diabetic retinopathy, rhinitis, ischemic-reperfusion injury, vascular restenosis, glomerulonephritis and gastrointestinal allergy.

13. A pharmaceutical composition for prevention or treatment of intestinal diseases, comprising an exosome which includes glucosamine, a glucosamine derivative or a salt thereof, as an active ingredient.

14. The pharmaceutical composition according to claim 13, wherein the intestinal diseases are any one of inflammatory bowel disease (IBD), irritable bowel syndrome (IBS), traveler's diarrhea, constipation, acute diarrhea, enteritis, gastroenteritis, abdominal pain or abdominal distension.

15. The pharmaceutical composition according to claim 14, wherein the inflammatory bowel disease is Crohn's disease, intestinal lesions involved in Behcet's disease, ulcerative colitis, hemorrhagic rectal ulcer and ileal cystitis.

16. A food composition for improving intestinal function or promoting bowel movement, comprising an exosome which includes glucosamine, a glucosamine derivate or a salt thereof, as an active ingredient.

17. A pharmaceutical composition for prevention or treatment of hair loss, comprising an exosome which includes glucosamine, a glucosamine derivative or a salt thereof, as an active ingredient.

18. A cosmetic composition for skin improvement, comprising an exosome which includes glucosamine, a glucosamine derivative or a salt thereof, as an active ingredient.

19. The cosmetic composition according to claim 18, wherein the skin improvement includes skin whitening, wrinkle improvement, elasticity enhancement, skin regeneration, skin moisturizing, anti-aging, alleviation of skin irritation, acne prevention or improvement, or atopic prevention or improvement effects.

20. A pharmaceutical composition for prevention or treatment of wounds, comprising an exosome which includes glucosamine, a glucosamine derivative or a salt thereof, as an active ingredient.

21. A screening method of a pharmaceutical composition or cosmetic composition comprising an exosome which includes glucosamine, a glucosamine derivative or a salt thereof, comprising:
(1) separating the exosome from cells; and
(2) measuring an expression level of the glucosamine, glucosamine derivative or salt thereof in the exosome.

22. The screening method according to claim 21, wherein, if the glucosamine, glucosamine derivative or salt thereof is present in the exosome or if the glucosamine, glucosamine derivative or salt thereof is included in the exosome in an amount of more than 0 and 20 wt.% or less in the exosome in the measuring step (2), further comprising a process of determining the exosome as: a pharmaceutical composition for anti-inflammation; a pharmaceutical composition for prevention or treatment of intestinal diseases; a pharmaceutical composition for prevention or treatment of hair loss; a pharmaceutical composition for prevention or treatment of wounds; a cosmetic composition for skin improvement; or an anti-inflammatory agent.

23. A method for prevention or treatment of inflammatory diseases, comprising administering an exosome, which includes glucosamine, a glucosamine derivative or a salt thereof, as an active ingredient to a subject suffering from inflammatory disease.

24. A use of the pharmaceutical composition for anti-inflammation according to any one of claims 10 to 12.

25. A method for prevention or treatment of intestinal diseases, comprising administering an exosome, which includes glucosamine, a glucosamine derivative or a salt thereof, as an active ingredient to a subject suffering from the intestinal disease.

26. A use of the pharmaceutical composition for prevention or treatment of intestinal diseases according to any one of claims 13 to 15.

27. A method for prevention or treatment of hair loss, comprising administering an exosome, which includes glucosamine, a glucosamine derivative or a salt thereof, as an active ingredient to a subject having hair loss.

28. A use of the pharmaceutical composition for prevention or treatment of hair loss according to claim 17.

29. A method for prevention or treatment of wounds, comprising administering an exosome, which includes glucosamine, a glucosamine derivative or a salt thereof, as an active ingredient to a wounded subject.

30. A use of the pharmaceutical composition for prevention or treatment of wounds according to claim 20.
